# EUROPÄISCHE PATENTSCHRIFT

(11) **EP 2 108 011 B1**
(45) Veröffentlichungstag und Bekanntmachung des Hinweises auf die Patenterteilung: **28.08.2013**
(21) Anmeldenummer: 08701190.4
(22) Anmeldetag: 02.01.2008
(51) Int. Cl.: C07C 209/78, C07C 209/84, C07C 211/50

(54) **VERFAHREN ZUR HERSTELLUNG VON DIPHENYLMETHANDIAMIN**
METHOD FOR THE PRODUCTION OF DIPHENYLMETHANE DIAMINE
PROCÉDÉ DE PRODUCTION DE DIPHÉNYLMÉTHANEDIAMINE

(30) Priorität: 08.01.2007 EP 07100242
(43) Veröffentlichungstag der Anmeldung: 14.10.2009
(73) Patentinhaber: BASF SE, 67056 Ludwigshafen (DE)
(72) Erfinder: WLOKA, Veronika, 68163 Mannheim (DE); MATTKE, Torsten, 67251 Freinsheim (DE); BREUNINGER, Daniel, 67240 Bobenheim-Roxheim (DE); SIEGERT, Markus, 69126 Heidelberg (DE)
(86) Internationale Anmeldenummer: PCT/EP2008/050002
(87) Internationale Veröffentlichungsnummer: WO 2008/083997

(56) Entgegenhaltungen:
- WO-A-99/40059
- WO-A-2005/007613
- DE-A1- 1 901 993
- DE-A1- 1 913 473
- US-A- 2 781 245

## Beschreibung

Die Herstellung von Diphenylmethandiamin (MDA) durch Umsetzung von Anilin mit Formaldehyd in Anwesenheit einer Säure ist bekannt und vielfach beschrieben. In der Praxis fällt das so hergestellte Diphenylmethandiamin stets im Gemisch mit höherkondensierten Polyphenylenpolymethylenpolyaminen an. Im folgenden wird unter "MDA" das Gemisch aus zweikernigem Diphenylmethandiamin und höherkondensierten Polyphenylenpolymethylenpolyaminen verstanden werden.

Das MDA wird in der Technik zumeist durch Umsetzung mit Phosgen zu Dipenylmethandiisocyanat MDI umgesetzt.

Für bestimmte Einsatzgebiete, beispielsweise als Vernetzer in Kunststoffen oder Lacken, kann auch reines 2-Kern-MDA eingesetzt werden.

In der Technik erfolgt die Herstellung des MDA, wie beschrieben, durch Umsetzung von Anilin mit Formaldehyd in Anwesenheit einer Säure. Als Säure wird üblicherweise Salzsäure eingesetzt. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben.

Durch die Variation des Verhältnisses von Säure zu Anilin und von Formaldehyd zu Anilin kann der Anteil des 2-Kern-Produkts im MDA je nach Wunsch eingestellt werden.

Ein Problem bei der Herstellung von MDA ist die Neutralisation. Zum einen ist die übliche Neutralisation mittels Natronlauge kostspielig. Zum anderen muss die dabei anfallende Sole kostspielig als Abwasser entsorgt werden.

Eine Möglichkeit, diese Schwierigkeiten zu umgehen, ist die Verwendung von heterogenen sauren Katalysatoren. So wird in WO 01/58847 ein Verfahren zur Herstellung von MDA beschrieben, bei dem Anilin mit Formaldehyd bei einem molaren Verhältnis von 1,7 bis 100 in Gegenwart fester, anorganischer, saurer Katalysatoren umgesetzt wird. Nachteilig bei dem dort beschriebenen Verfahren sind die ungenügende Standzeit des Katalysators durch Desaktivierung infolge Belagsbildung mit Oligomeren, die Titration der Säuregruppen des Katalysators mit im Anilin enthaltenen oder während der Reaktion gebildeten sekundären Aminen, wie N-Methylaminen, sowie die hohen Kosten für den Katalysator und für durch Wechsel und Regenerierung des Katalysators bedingte Stillstände.

In WO 2005/007613 wird ein Verfahren zur Herstellung von MDA beschrieben, bei dem der saure Katalysator durch ein Adsorptionsmittel entfernt wird. Durch Regeneration kann die Säure aus dem Adsorptionsmittel zurückgewonnen und wieder eingesetzt werden. Nachteilig ist auch bei diesem Verfahren die geringe Standzeit des Adsorptionsmittels auf Grund der Belegung der Oberfläche.

Die Verwendung von Oxiden und/oder Hydroxiden von anderen Metallen, insbesondere Erdalkalimetallen zur Neutralisation der Reaktionsmischung scheiterte zumeist daran, dass es dabei zu einer verstärkten Feststoffbildung kommt, die zu Störungen in der Antage führen können.

DE 1913473 beschreibt ein Verfahren zur Herstellung von 11DA, wobei die Säure mit Ammoniak neutralisiert wird.

Es war die Aufgabe der vorliegenden Erfindung, ein Verfahren zur Herstellung von MDA zu entwickeln, bei dem eine einfache, kostengünstige und betriebssichere Abtrennung des sauren Katalysators möglich ist, ohne dass es zu Nachteilen bei der Verfahrensführung kommt.

Die Aufgabe konnte überraschenderweise dadurch gelöst werden, dass als Neutralisationsmittel Ammoniak eingesetzt wird, der in einem weiteren Verfahrensschritt durch Behandlung mit dem Oxid oder Hydroxid eines Erdalkalimetalls zurückgewonnen wird.

Gegenstand der Erfindung ist demzufolge ein Verfahren zur Herstellung von Diphenylmethandiamin, umfassend die Schritte
a) Umsetzung von Anilin mit Formaldehyd in Anwesenheit einer Säure,
b) Neutralisation der Säure mit Ammoniak,
c) Trennung des Reaktionsgemisches aus Schritt b) in eine wässrige und eine organische Phase,
d) Behandlung der in Schritt c) erhaltenen wässrigen Phase mit einem Oxid oder Hydroxid eines Erdalkalimetalls,
e) Abtrennung des in Schritt d) erhaltenen Ammoniaks.

Vorzugsweise wird als Säure eine Mineralsäure, insbesondere Salzsäure, eingesetzt.

Prinzipiell können in Schritt d) Oxide oder Hydroxide der Erdalkalimetalle eingesetzt werden. Vorzug hat Calciumoxid und/oder Calciumhydroxid, da diese gut verfügbar sind und die entstehenden Abprodukte unproblematisch zu handhaben und zu entsorgen sind.

Die Herstellung des MDA in Schritt a) erfolgt, wie oben beschrieben, durch Umsetzung von Anilin mit Formaldehyd in Gegenwart von Säuren als Katalysatoren. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben.

An Stelle von oder im Gemisch mit Formaldehyd kann auch mindestens eine Formaldehyd abspaltende Verbindung eingesetzt werden. Insbesondere wird der Formaldehyd als wässrige Formalinlösung, alkoholische Formalinlösung, Halbacetal, Methylenimin eines primären Amins oder N,N`-Methylendiamin eines primären oder sekundären Amins sowie Paraformaldehyd eingesetzt.

Das erfindungsgemäße Verfahren kann kontinuierlich, halbkontinuierlich oder batchweise, vorzugsweise kontinuierlich oder halbkontinuierlich, durchgeführt werden.

Bei der kontinuierlichen Fahrweise werden die Reaktanden in dem gewünschten Verhältnis zueinander in einen Reaktor eindosiert und diesem Reaktor eine dem Zustrom gleiche Menge an Reaktionsprodukt entnommen. Als Reaktoren kommen beispielsweise Rohrreaktoren zum Einsatz. Bei der batchweisen oder halbkontinuierlichen Fahrweise werden die Reaktanden in einen vorzugsweise mit einem Rührer und/oder einem Umpumpkreis versehenen Batchreaktor dosiert, aus dem das ausreagierte Reaktionsprodukt entnommen und der Aufarbeitung zugeführt wird.

Das erfindungsgemäße Verfahren wird vorzugsweise bei einem molaren Verhältnis von Anilin zu Formaldehyd größer 2 durchgeführt. Das molare Verhältnis von Säure zu Anilin ist vorzugsweise größer 0,05. Bei diesen Verhältnissen kommt es zu einer verstärkten Bildung der jeweiligen Zweikernprodukte in der Reaktionsmischung.

Die Reaktion wird vorzugsweise bei einer Temperatur im Bereich zwischen 0 und 200 °C, vorzugsweise zwischen 20 und 150 °C und insbesondere zwischen 40 und 120 °C durchgeführt. Es hat sich gezeigt, dass mit der Erhöhung der Temperatur der Anteil der 2,2'- und 2,4'-Isomeren im Reaktionsprodukt ansteigt.

Der Druck bei der Umsetzung beträgt 0,1 - 50, bevorzugt 1 - 10 bar absolut.

Bei der batchweisen und halbkoninuierlichen Durchführung der Reaktion kann nach der vollständigen Dosierung der Einsatzstoffe das Reaktionsgemisch einer sogenannten Alterung unterzogen werden. Dazu wird das Reaktionsgemisch im Reaktor belassen oder in einen anderen, vorzugsweise gerührten Reaktor überführt. Dabei liegt die Temperatur des Reaktionsgemisches vorzugsweise über 75 °C, insbesondere in einem Bereich zwischen 110 und 150 °C.

An die Herstellung in Schritt a) schließt sich die Neutralisation b) des Reaktionsgemischs an. Dazu wird der Reaktionsmischung Ammoniak zugesetzt. Dieses kann der Reaktionsmischung gasförmig, gegebenenfalls mit Wasser gesättigt, als wässrige Ammoniaklösung oder als Mischung beider Phasen zugeführt werden. Die Zusammenführung des Ammoniaks mit der Reaktionsmischung erfolgt üblicherweise in einem geeigneten Mischapparat wie einem Rührkessel, einem Rohr, das gegebenenfalls mit statischen Mischelementen versehen ist, oder anderen Apparaten. Die Zugabe basischen Ammoniaks bewirkt eine Neutralisation der Reaktionsmischung und dadurch die Bildung zweier nichtmischbarer Phasen, der wässrigen und der organischen Phase. Die Neutralisation erfolgt bei einer mittleren Temperatur von 40 bis 120 °C und einem Druck von 1 bis 10 bar absolut.

Das Gemisch aus Schritt b) liegt, wie beschrieben, in einer organischen und einer wässrigen Phase vor. Diese Phasen werden in Schritt c) getrennt. Die Phasen können beispielsweise durch Dekantieren voneinander getrennt werden. Danach werden die jeweiligen Phasen separat aufgearbeitet.

Die wässrige Phase, die im wesentlichen aus Wasser, dem darin gelösten Ammoniumsalz der als Katalysator eingesetzten Säure, sowie Spuren der Einsatzstoffe Anilin und Formaldehyd und auch des Endprodukts MDA besteht, wird in Schritt d) mit dem Oxid und/oder Hydroxid eines Erdalkalimetalls behandelt. Wie oben beschrieben, werden vorzugsweise die entsprechenden Verbindungen des Calciums, meist in Form von Kalkmilch bzw. Löschkalk, zum Einsatz kommen. Dabei wird das Ammoniumsalz unter Bildung von Ammoniak zersetzt. Dieser Verfahrensschritt ist als Teilschritt aus dem SOLVAY-Prozess zur Herstellung von Soda bekannt. Die Abtrennung des Ammoniaks erfolgt bevorzugt destillativ oder durch Strippen mit Wasserdampf oder einem Inertgas.

Die ammoniakreiche Gasphase wird gegebenenfalls in weiteren Schritten, wie Trocknung durch Adsorption oder Auskondensieren von Wasserdampf aufkonzentriert und gereinigt und kann anschließend wieder der Neutralisation b) zugeführt werden.

In einer besonderen Ausführungsform wird das Wasserdampf enthaltende Gas über Calciumoxid, auch als Branntkalk bezeichnet, geführt. Dabei wird einerseits das Gas getrocknet und zum zweiten der Branntkalk in Calciumhydroxid, sogenannten Löschkalk, überführt, der wiederum der Ersetzung des Ammoniumsalzes in Schritt d) zugeführt wird.

Die nach der Entfernung des Ammoniaks verbleibende ammoniakarme flüssige Phase kann, gegebenenfalls nach weiterer Aufkonzentration bzw. Aufreinigung, als Abwasser entsorgt werden.

Die in Schritt c) abgetrennte organische Phase, die überwiegend aus MDA mit Resten von Wasser, Ammoniak und den Einsatzprodukten für die Herstellung des MDA besteht, wird ebenfalls aufgearbeitet. Dies erfolgt beispielsweise durch ein- oder mehrmaliges Waschen mit Wasser oder bevorzugt durch mehrfache Destillation zur Abtrennung von beispielsweise Anilin und Wasser.

Das nach dem erfindungsgemäßen Verfahren hergestellte MDA wird üblicherweise mit Phosgen zu MDI umgesetzt. Derartige Verfahren sind allgemein bekannt und vielfach beschrieben, beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059 oder WO 99/54289.

Dazu wird üblicherweise das MDA und gegebenenfalls das Phosgen in einem inerten Lösungsmittel gelöst und zur Reaktion gebracht. Als Lösungsmittel werden vorzugsweise inerte organische, insbesondere aromatische Lösungsmittel, wie Toluol oder halogenierte aromatische Verbindungen, wie Monochlorbenzol, eingesetzt.

Das genannte Verfahren kann in üblichen Reaktoren, beispielsweise Rührkesseln, Rührkesselkaskaden, Kolonnen und/oder Rohrreaktoren bei bekannten Temperaturen von z. B. 50 bis 150 °C, bevorzugt 70 bis 120 °C, besonders bevorzugt 70 bis 100 °C und einem Druck von 0,5 bis 10 bar, bevorzugt 0,8 bis 5 bar, besonders bevorzugt 0,8 bis 1,5 bar in einer oder mehreren Stufen durchgeführt werden.

Beispielsweise kann die Phosgenierung durchgeführt werden durch eine zweistufige Umsetzung in Gegenwart mindestens eines inerten organischen Lösungsmittels, wobei die erste Stufe der Phosgenierung in einem statischen Mischer und die zweite Stufe der Phosgenierung in einem Verweilzeitapparat durchgeführt werden.

Das durch die Phosgenierung hergestellte Roh-MDI kann durch übliche Verfahren, beispielsweise Destillation, gereinigt werden. Bevorzugt kann in einem ersten Reinigungsvorgang Phosgen und gegebenenfalls Lösungsmittel, bevorzugt weitgehend, besonders bevorzugt vollständig aus dem Reaktionsgemisch der Phosgenierung, d. h. dem Roh-MDI entfernt werden.

Bevorzugt kann anschließend gewünschtes monomeres MDI, beispielsweise 2,2'-, 2,4'- und/oder 4,4'-MDI und/oder Gemische enthaltend mindestens zwei diese Isomere, durch ein geeignetes Verfahren, bevorzugt durch Destination, beispielsweise bei Drücken von 2 bis 50 mbar, bevorzugt 2 bis 20 mbar, und Temperaturen von 150 bis 250 °C, bevorzugt 180 bis 230 °C, und/oder bevorzugt durch Kristallisation, beispielsweise fraktionierte Kristallisation, abgetrennt werden.

In einer besonderen Ausführungsform des Verfahrens zur Herstellung von MDI kann aus dem Roh-MDA das Zwei-Kern-Produkt abgetrennt und mittels Gasphasenphosgenierung, wie beispielsweise EP 570 799 beschrieben, zu Zwei-Kern-MDI umgesetzt werden.

Das so hergestellte MDI kann insbesondere mit Verbindungen mit mindestens zwei aktiven Wasserstoffatomen zu Polyurethanen umgesetzt werden.

Das erfindungsgemäße Verfahren erlaubt eine kostengünstige und betriebssichere Aufarbeitung von MDA. Es kommt zu keiner Schädigung des MDA. Das eingesetzte Ammoniak kann vollständig vom Reaktionsprodukt abgetrennt werden. Durch die Kreislaufführung des Ammoniaks werden Produktverluste vermieden. Die entstehenden wässrigen Salzlösungen können problemlos entsorgt werden Die Erfindung soll an dem nachfolgenden Beispiel näher erläutert werden.

### Beispiel :

In einer Laborapparatur mit Rührwerk wurde 1 kg Anilin mit 0,5 kg einer 30 Gew-%igen Salzsäure gemischt und die Mischung auf 50 °C temperiert. Über 1 Stunde wurde kontinuierlich insgesamt 0,4 kg einer 40 Gew-%igen wässrigen Formaldehyd-Lösung dosiert. Danach wurde die Mischung auf 100 °C erwärmt und 12 Stunden bei dieser Temperatur gerührt. In dieser Zeit erfolgte die Umsetzung des Anilins mit dem Formaldehyd zu MDA.

Danach wurde der Mischung etwa 350 g einer 20 Gew%igen Ammoniaklösung mit 50 °C zudosiert und die Mischung intensiv 30 Minuten gerührt. Anschließend wurde der Rührer ausgeschaltet. Es erfolgte eine Trennung der Reaktionsmischung in eine wässrige und eine organische Phase. Nach erfolgter Phasentrennung wurden die Phasen getrennt aus der Laborapparatur abgezogen.

Die abgezogene wässrige Phase enthielt etwa 18,5 Gew.-% Ammoniumchlorid.

Zu 1 kg der wässrigen Phase wurden in einer temperierten Laborapparatur 150 g Calciumhydroxid (Löschkalk) gegeben. Der dabei entstehende Wasser enthaltende gasförmige Ammoniak wurde über einen mit Calciumoxid gefüllten Trockenturm geführt und so getrocknet. Das getrocknete Ammoniak konnte erneut zur Neutralisation eingesetzt werden.

Nach vollständiger Entgasung verblieb in der Apparatur eine etwa 20%ige wässrige Lösung von Calciumchlorid zur Entsorgung.

## Patentansprüche

1. Verfahren zur Herstellung von Diphenylmethandiamin, umfassend die Schritte
a) Umsetzung von Anilin mit Formaldehyd in Anwesenheit einer Säure,
b) Neutralisation der Säure mit Ammoniak,
c) Trennung des Reaktionsgemisches aus Schritt b) in eine wässrige und eine organische Phase,
d) Behandlung der in Schritt c) erhaltenen wässrigen Phase mit einem Oxid oder Hydroxid eines Erdalkalimetalls,
e) Abtrennung des in Schritt d) erhaltenen Ammoniaks.

2. Verfahren nach Anspruch 1, **dadurch gekennzeichnet, dass** als Säure eine Mineralsäure eingesetzt wird.

3. Verfahren nach Anspruch 1, **dadurch gekennzeichnet, dass** als Säure Salzsäure eingesetzt wird.

4. Verfahren nach Anspruch 1, **dadurch gekennzeichnet, dass** in Schritt d) als Oxid oder Hydroxid eines Erdalkalimetalls Calciumoxid und/oder Calciumhydroxid eingesetzt wird.

5. Verfahren nach Anspruch 1, **dadurch gekennzeichnet, dass** in Schritt d) als Hydroxid eines Erdalkalimetalls Calciumhydroxid eingesetzt wird.

6. Verfahren nach Anspruch 1, **dadurch gekennzeichnet, dass** das in Schritt e) erhaltene Ammoniak in den Schritt b) zurückgeführt wird.

7. Verfahren nach Anspruch 1, **dadurch gekennzeichnet, dass** die in Schritt c) erhaltene organische Phase gereinigt und zu Diphenylmethandiamin aufgearbeitet wird.

8. Verfahren nach Anspruch 1, **dadurch gekennzeichnet, dass** das geleinigte Diphenylmethandiamin mit Phosgen zu Diphenylmethandlisocyanat umgesetzt wird.

## Claims

1. A process for preparing diphenylmethanediamine, comprising the steps of
a) reacting aniline with formaldehyde in the presence of an acid,
b) neutralizing the acid with ammonia,
c) separating the reaction mixture from step b) into an aqueous phase and an organic phase,
d) treating the aqueous phase obtained in step c) with an oxide or hydroxide of an alkaline earth metal,
e) separating off the ammonia obtained in step d).

2. The process according to claim 1, wherein the acid used is a mineral acid.

3. The process according to claim 1, wherein the acid used is hydrochloric acid.

4. The process according to claim 1, wherein the oxide or hydroxide of an alkaline earth metal that is used in step d) is calcium oxide and/or calcium hydroxide.

5. The process according to claim 1, wherein the hydroxide of an alkaline earth metal that is used in step d) is calcium hydroxide.

6. The process according to claim 1, wherein the ammonia obtained in step e) is passed back into step b).

7. The process according to claim 1, wherein the organic phase obtained in step c) is purified and worked up to give diphenylmethanediamine.

8. The process according to claim 1, wherein the purified diphenylmethanediamine is reacted with phosgene to give diphenylmethane diisocyanate.

## Revendications

1. Procédé pour la préparation de diphénylméthanediamine, comprenant les étapes
a) transformation d'aniline avec du formaldéhyde en présence d'un acide,
b) neutralisation de l'acide par de l'ammoniaque,
c) séparation du mélange réactionnel de l'étape b) en une phase aqueuse et une phase organique,
d) traitement de la phase aqueuse obtenue dans l'étape c) avec un oxyde ou un hydroxyde d'un métal alcalino-terreux,
e) séparation de l'ammoniaque obtenue dans l'étape d).

2. Procédé selon la revendication 1, **caractérisé en ce qu'**on utilise, comme acide, un acide minéral.

3. Procédé selon la revendication 1, **caractérisé en ce qu'**on utilise, comme acide, de l'acide chlorhydrique.

4. Procédé selon la revendication 1, **caractérisé en ce qu'**on utilise, dans l'étape d), comme oxyde ou hydroxyde d'un métal alcalino-terreux, de l'oxyde de calcium et/ou de l'hydroxyde de calcium.

5. Procédé selon la revendication 1, **caractérisé en ce qu'**on utilise, dans l'étape d), comme hydroxyde d'un métal alcalino-terreux, de l'hydroxyde de calcium.

6. Procédé selon la revendication 1, **caractérisé en ce que** l'ammoniaque obtenue dans l'étape e) est recyclée dans l'étape b).

7. Procédé selon la revendication 1, **caractérisé en ce que** la phase organique obtenue dans l'étape c) est purifiée et transformée en diphénylméthanediamine.

8. Procédé selon la revendication 1, **caractérisé en ce que** la diphénylméthanediamine purifiée est transformée avec du phosgène en diphénylméthanediisocyanate.
